(19) Europäisches Patentamt
European Patent Office
Office européen des brevets

(11) Numéro de publication: **0 274 967**
**A2**

(12) # DEMANDE DE BREVET EUROPEEN

(21) Numéro de dépôt: **87420332.6**

(22) Date de dépôt: **14.12.87**

(51) Int. Cl.⁴: **C07D 207/452**, C08F 222/40, C08G 73/12, C08G 77/42

(30) Priorité: **17.12.86 FR 8617916**

(43) Date de publication de la demande: **20.07.88 Bulletin 88/29**

(84) Etats contractants désignés: **AT BE CH DE ES FR GB IT LI LU NL SE**

(71) Demandeur: **RHONE-POULENC CHIMIE**
**25, quai Paul Doumer**
**F-92408 Courbevoie Cédex(FR)**

(72) Inventeur: **Barthelemy, Pascal**
**21, rue de la Part-Dieu**
**F-69003 Lyon(FR)**
Inventeur: **Crochemore, Michel**
**3, rue des Lilas Domaine de Gilbertain**
**F-69630 Chaponost(FR)**

(74) Mandataire: **Trolliet, Maurice et al**
**RHONE-POULENC INTERSERVICES Service Brevets Chimie Centre de Recherches des Carrières B.P. 62**
**F-69192 Saint-Fons Cédex(FR)**

(54) **Nouveaux composés à base de maléimides et nouvelles compositions thermodurcissables les contenant.**

(57) La présente invention concerne de nouveaux composés comprenant un mélange de monomaléimides.

Elle concerne également de nouvelles compositions thermodurcissables à groupements imides possédant des propriétés mécaniques améliorées.

Les nouveaux composés comprennent un mélange de N-(méth)allyloxyphénylmaléimides avec ses dérivés de substitution (méth)allylés sur le cycle benzénique.

Les nouvelles compositions comprennent essentiellement le produit de réaction entre :

(a) un ou plusieurs bis-imides ;
(b) un composé répondant à la définition donnée ci-dessus ;
(c) éventuellement un composé organosilicique hydroxylé ; et
(d) un composé imidazole.

Ces compositions peuvent être utilisées pour la réalisation de revêtements, de collages, de stratifiés et matériaux composites renforcés.

EP 0 274 967 A2

# NOUVEAUX COMPOSES A BASE DE MALEIMIDES ET NOUVELLES COMPOSITIONS THERMODURCISSABLES LES CONTENANT

La présente invention concerne de nouveaux composés comprenant un mélange de monomaléimides N-substitués.

Elle concerne également de nouvelles compositions thermodurcissables à base desdits composés possédant des propriétés mécaniques améliorées.

Les maléimides N-substitués sont une famille connue de composés chimiques et les bis-maléimides N,N'-disubstitués sont notamment utilisés pour la préparation de polymères thermodurcissables, les polybismaléimides.

Des monomaléimides sont également connus. Ainsi le brevet américain No. 2.444.536 décrit un procédé de préparation de N-arylmaléimides.

Certains monomaléimides peuvent être utilisés en agrochimie comme insecticides ou fongicides. D'autres peuvent servir à préparer des polymères réticulables sous l'influence de la lumière.

Les monomaléimides peuvent également être employés en mélanges avec des bis-maléimides pour la production de polymères thermodurcissables.

Un des objets de la présente invention consiste en de nouveaux composés comprenant :

- un mélange d'un monomère de formule :

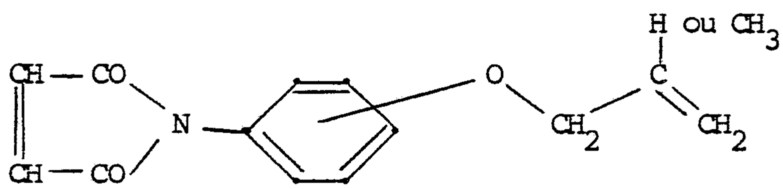

(I)

dans laquelle le radical allyloxy ou méthallyloxy est en position ortho, méta ou para par rapport à l'atome de carbone du cycle benzénique relié à l'azote.

- avec :

. au moins un dérivé monosubstitué de formule :

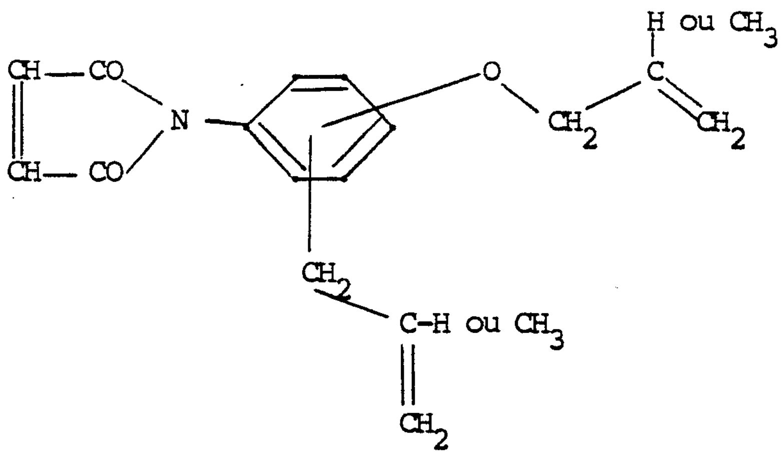

(II)

. et éventuellement un ou plusieurs dérivés disubstitués de formule :

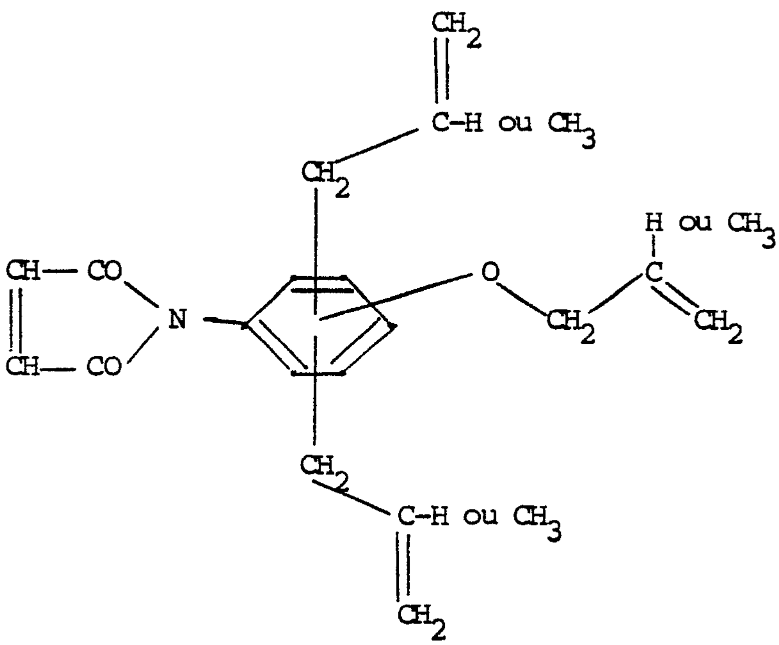

(III)

Dans le composé précité les proportions des divers constituants du mélange des produits de formules (I), (II) et éventuellement (III) peuvent varier dans des larges limites. De manière générale, les proportions des constituants sont choisies entre les limites suivantes (exprimant le pourcentage pondéral de chacun des consituants dans le mélange) :

- au moins 30 %, et de préférence de 50 % à 80 % de N-(méth)allyloxyphénylmaléimide de formule (I),
- de 5 % à 50 % et de préférence de 10 % à 35 % de dérivé(s) mono-(méth)allyl substitué(s) de formule (II),
-et de 0 % à 20 % et de préférence de 0 % à 15 % de dérivé(s) di-(méth)allyl substitué(s) de formule (III),

la somme des constituants devant dans chaque cas être égale à 100 % en poids.

Les composés selon l'invention comprenant des mélanges à base de N-(méth)allyloxyphénylmaléimide et de dérivé(s) de substitution (méth)allylé(s) peuvent être obtenus par exemple en mélangeant les ingrédients de formules (I), (II) et éventuellement (III) préparés séparément.

Les maléimides de formule (I) peuvent être notamment préparés à partir des aminophénols (ortho, méta ou para), selon la réaction de Claisen.

On peut par exemple faire réagir l'aminophénol, dont on a préalablement bloqué la fonction amine par réaction avec l'anhydride acétique pour former l'acétamidophénol, avec selon le cas un halogénure d'allyle (le plus souvent le bromure) ou de méthallyle en solution dans l'acétone et en présence de carbonate dipotassique. La fonction amine est ensuite régénérée par hydrolyse.

On prépare ensuite de manière classique la maléimide correspondant en faisant réagir en solution

l'allyloxyaniline ou la métallyloxyaniline obtenue précédemment avec l'anhydride maléique, en présence d'anhydride acétique, de triéthylamine et d'un sel de nickel (acétate de nickel en particulier).

On obtient ainsi le N-allyloxyphényl-maléimide ou le N-méthallyloxyphényl-maléimide.

Le N-(allyloxy-4-phényl)maléimide est un solide de couleur jaune moutarde ayant un point de fusion de 103°C environ.

. L'analyse RMN est en accord avec la structure suivante :

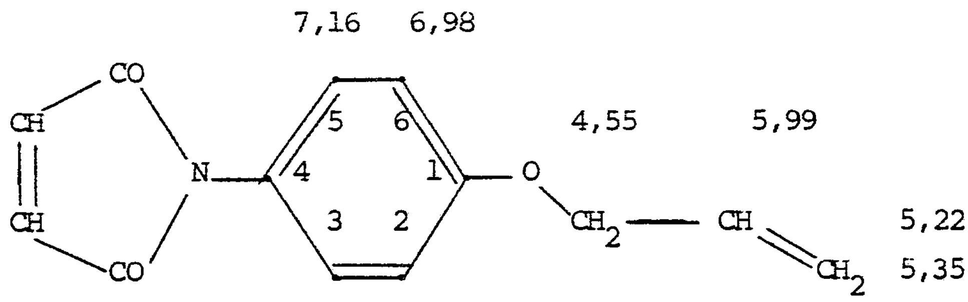

RMN 1H ; solvant : DMSO d6 ; référence : hexaméthyldisiloxane (HMDS)

7,16 (2H,m) : H 3,5 ;

7,10 (2H,s) : maléimido ;

6,98 (2H,m) : H 2,6 ;

5,99 (1H,m) : -CH= ;

5,35 et 5,22 (2H,dd) : $=CH_2$ ;

4,55 (2H,d) : $OCH_2$.

Le N-(allyloxy-3 phényl)maléimide est un liquide jaune orange visqueux, qui cristallise lentement à température ambiante et qui bout à 150°C environ sous une pression 20 Pa.

L'analyse RMN est en accord avec la structure suivante :

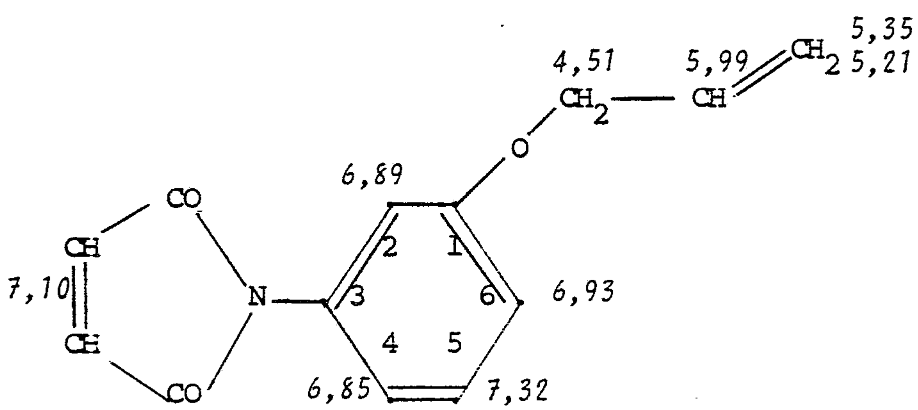

RMN 1H ; solvant : DMSO d6 ; référence : HMDS

6,85, 6,89 et 6,93 (3H,m) : H4, H2 et H6 ;

7,10 (2H,s) : maléimido ;

7,32 (1H,t) : H5 ;

5,99 (1H,m) : -CH= ;

5,35 et 5,21 (2H,dd) : $=CH_2$ ;

4,51 (2H,d) : $OCH_2$.

Le N-(allyloxy-2 phényl)maléimide est un solide cristallisé jaune clair, ayant un point de fusion d'environ 82°C et un point d'ébullition de 148°C à 155°C sous une pression de 20 Pa.

L'analyse RMN est en accord avec la structure suivante :

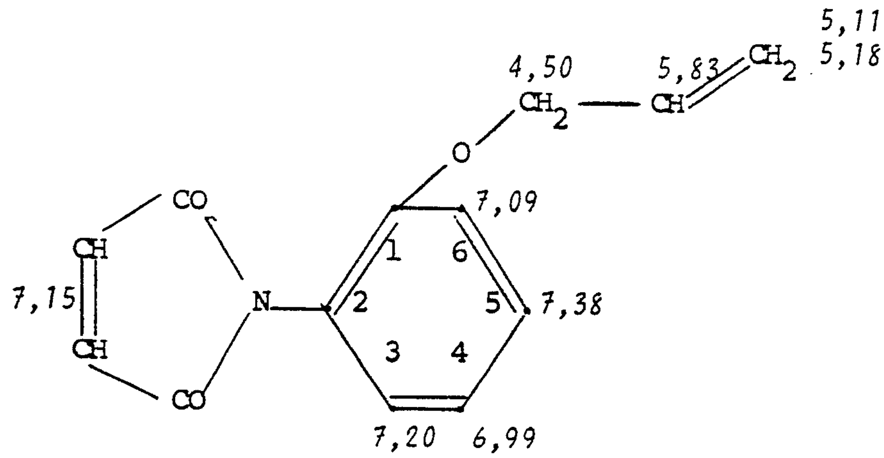

RMN 1H ; solvant : DMSO d6 ; référence : HMDS

7,38 (1H,dt) : H5 ;
7,20 (1H,dd) : H3 ;
7,15 (2H,s) : maléimido ;
7.09 (1H,dd) : H6 ;
6,99 (1H,dt) : H4 ;
5,83 (1H,m) : -CH = ;
5,18 et 5,11 (2H,dd) : $=CH_2$ ;
4,50 (2H,d) : $OCH_2$.

Le N-(méthallyloxy-4 phényl) maléimide est un solide de couleur beige ayant un point de fusion de 64°C.

L'analyse RMN est en accord avec la structure suivante :

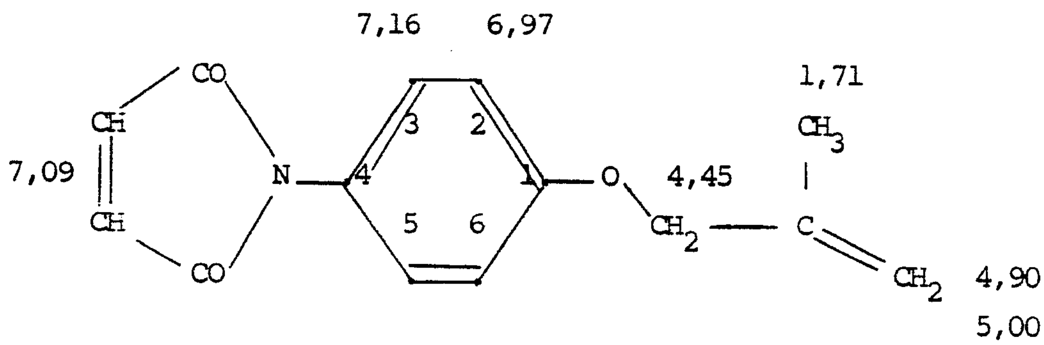

RMN 1H ; solvant : DMSO d6 ; référence : HMDS

7,16 (2H,d) : H 3,5 ;
7.09 (2H,s) : maléimido ;
6,97 (2H,d) : H 2,6 ;
4,90 et 5,00 (1H,s) : $CH_2=$ ;
4,45 (2H,s) : $OCH_2$ ;
1,71 (3H,s) : $CH_3$.

Le N-(méthallyloxy-3 phényl) maléimide est un solide de couleur beige ayant un point de fusion de 39°C.

L'analyse RMN est en accord avec la structure suivante :

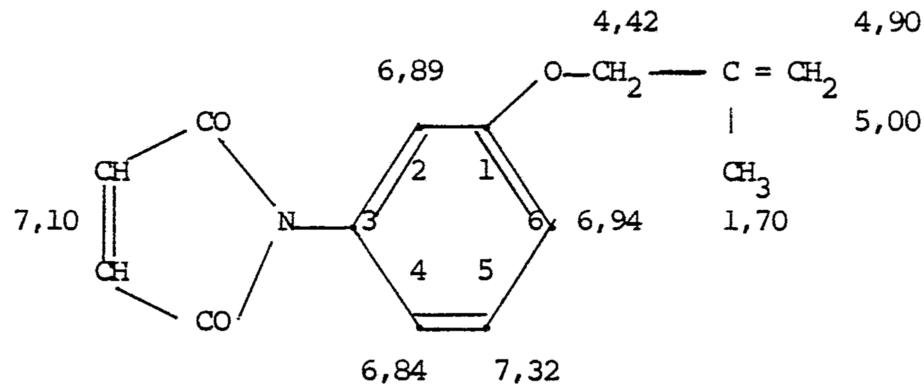

RMN 1H ; solvant : DMSO d6 ; référence : HMDS

7,32 (1H,t) : H5 ;
7,10 (2H,s) : maléimido ;
6,94 (1H,d) : H6 ;
6,89 (1H,s) : H2 ;
6,84 (1H,d) : H4 ;
4,90 et 5,00 (1H,1) : $CH_2$ = ;
4,42 (2H,s) : $OCH_2$ ;
1,70 (3H,s) : $CH_3$.

Le N-(méthallyloxy-2 phényl) maléimide est un solide de couleur beige ayant un point de fusion de 96°C.

L'analyse RMN est en accord avec la structure suivante :

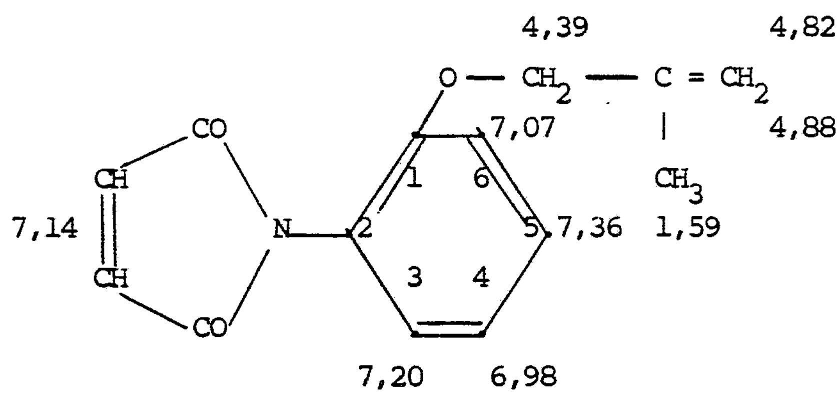

RMN 1H ; solvant : DMSO d6 ; référence : HMDS

7,36 (1H,t) : H5 ;
7,20 (1H,d) : H3 ;
7,14 (2H,s) : maléimido ;
7,07 (1H,d) : H6 ;
6,98 (1H,t) : H4 ;
4,82 et 4,88 (1H,s) : $CH_2$= ;
4,39 (2H,s) : $OCH_2$ ;
1,59 (3H,s) : $CH_3$.

Conformément à une modalité préférée de la présente invention, le composé comprenant un mélange de N-(méth)allyloxyphénylmaléimide de formule (I) avec un ou plusieurs dérivés de substitution (méth)-allylés de formule(s) (II) et éventuellement (III) se présente sous la forme du produit brut obtenu par mise en oeuvre du procédé original ci-après défini.

Ce procédé est caractérisé en ce qu'il consiste à réaliser les 3 étapes suivantes qui sont enchaînées dans le même réacteur :

-la première étape consiste à faire réagir en milieu solvant un aminophénol avec de l'anhydride maléique en opérant à une température allant de 20° C à 200° C, pendant une durée allant, selon la température

choisie, de 30 minutes à 2 heures et elle conduit à un premier milieu réactionnel comprenant un acide N-(hydroxylphényl)maléamique ;

-la deuxième étape consiste à réaliser une réaction de (méth)allylation de l'acide précité en faisant réagir le premier milieu réactionnel précité avec un halogénure de (méth)allyle en opérant à un pH qui doit être ajusté et maintenu à une valeur constante comprise entre 7 et 14 par addition d'une quantité déterminée d'une solution aqueuse alcaline et à une température allant de 40° C à 150° C et elle conduit, après acidification et élimination de la phase aqueuse, à un second milieu réactionnel organique comprenant un acide N-[(méth)allyloxyphényl]maléamique, un ou plusieurs acides N-[(méth)allyloxy, (méth)allyphényl]-maléamiques et éventuellement unou plusieurs acides N-[(méth)allyloxy,di(méth)allylphényl]maléamiques :

-la troisième étape consiste à réaliser une réaction de cyclisation des acides maléamiques précités en faisant réagir le second milieu réactionnel précité avec un anhydride d'acide carboxilique inférieur, en présence d'une amine tertiaire et éventuellement d'un catalyseur, puis à éliminer le solvant de la réaction et elle conduit à un produit brut de réaction qui est un composé comprenant un mélange formé de : au moins 30 % en poids et de préférence de 50 % à 80 % en poids de N-(méth)allyloxyphénylmaléimide, de 5 % à 50% en poids et de préférence de 10 % à 35 % en poids d'un ou plusieurs N-[(méth)allyloxy, (méth)-allylphényl]maléimides et de 0% à 20% en poids et de préférence de 0 % à 15 % en poids d'un ou plusieurs N-[méth)allyloxy, di(méth)allylphényl]maléimides.

Les 3 étapes qui viennent d'être décrites sont enchaînées dans un solvant unique pour une plus grande simplicité du procédé, mais il est possible d'opérer un changement de solvant, au cours de telle ou telle étape, sans inconvénient. Le choix du solvant peut être très large, mais, comme la deuxième étape est réalisée en milieu biphasique hydro-organique, il peut être souhaitable de mettre en oeuvre un solvant organique non miscible à l'eau, ce qui simplifie considérablement le traitement de la masse réactionnelle. Parmi les solvants miscibles ou non miscibles à l'eau utilisables, on donnera en fait la préférence à ceux qui dissolvent, dans les conditions de température adoptées pour la synthèse, l'aminophénol de départ. Parmi ces solvants, on peut citer par exemple : les alcools (comme par exemple : méthanol, éthanol, butanol) ; les cétones (comme par exemple : acétone, méthyléthylcétone, méthylisobutylcétone) ; les nitriles (comme par exemple : benzonitrile, propionitrile, acétonitrile) ; les esters (comme par exemple : acétate d'éthyle, de butyle) ; des solvants aromatiques (comme par exemple : anisole, chlorobenzène) ; des hydrocarbures halogènés (comme par exemple :chloroforme, dichlorométhane, dichloroéthane).

S'agissant de la première étape du procédé, on peut dire que la concentration des réacitfs de départ dans le solvant utilisé n'est pas critique. On n'a pas intérêt tout de même ni à trop diluer pour des raisons de productivité, ni à trop concentrer pour avoir une bonne agitabilité. Dans cette première étape l'anhydride maléique est utilisé en quantités au moins égales à une mole par mole d'aminophénol ; on utilise généralement des quantités plus importantes qui sont de l'ordre de 1,01 à 1,5 moles par mole d'aminophénol. De plus la température se situe de préférence entre 40° C et 60° C.

S'agissant de la deuxième étape, on commence par ajouter dans le milieu réactionnel la quantité d'une solution aqueuse alcaline, par exemple d'une solution aqueuse de NaOH, nécessaire d'une part pour salifier l'acide N-(hydroxyphényl) maléamique et d'autre part pour obtenir le pH désiré. Le pH sera maintenu constant pendant toute la réaction par ajout de soude ; de manière préférentielle, le pH est ajusté et maintenu à une valeur constante comprise entre 10 et 12. La réaction d'allylation est réalisée de préférence avec le bromure ou le chlorure de (méth)allyle. La quantité d'halogénure de (méth)allyle est de l'ordre de 1,5 à 10 moles par groupement molaire OH phénolique et, de préférence, de l'ordre de 2 à 4. L'excès de ce réactif peut être récupéré en fin d'opération et recyclé dans une opération suivante. Le temps de coulée de l'halogénure de (méth)allyle n'est pas critique et il peut se situer dans l'intervalle allant de 1 heure à 5 heures, et, de préférence, allant de 2 heures à 4 heures. Dans cette deuxième étape, la température se situe de préférence entre 60° C et 100° C. A noter qu'en fin d'étape la phase aqueuse est acidifiée jusqu'à un pH environ égal à 1 par des acides habituels, de préférence des oxyacides ou des hydracides minéraux. La couche aqueuse est éliminée et on laisse dans le réacteur la couche organique.

S'agissant de la troisième étape du procédé, on utilise, avantageusement comme anhydride d'acide carboxylique inférieur, l'anhydride acétique en quantités au moins égales à une mole par groupement molaire HOOC-CH = CH-CO-NH-à cycliser. On utilise généralement des quantités plus importantes qui sont de l'ordre de 1,05 à 1,5 moles par groupement maléamique.

Parmi les amines tertiaires convenables, on peut citer notamment les trialkylamines ainsi que les N,N-dialkylanilines dans lesquelles les radicaux alkyles ont de 1 à 12 atomes de carbone. La triéthylamine et la N,N-diméthyl-aniline sont d'un emploi avantageux. Les quantités d'amine tertiaire se situent entre 0,05 et 2 moles par groupement molaire HOOC-CH CH-CO-NH-.

En tant que catalyseurs, on peut utiliser par exemple des sels de nickel d'acides carboxyliques, éventuellement hydratés ainsi que les formes chélatées de ce métal. L'acétate et l'acétylacétonate

conviennent particulièrement bien. Ces catalyseurs sont employés en quantités très faibles, de l'ordre de 0,05 à 1,5 g par groupement molaire HOOC-CH = = CH-CO-NH-et, de préférence, de l'ordre de 0.1 à 0.8 g.

Dans cette troisième étape, la température n'est pas critique et n'a une incidence que sur la cinétique de la réaction. Cette température peut être comprise par exemple entre 40° C et 150° C et, de préférence, entre 60° C et 80° C. En fin de cette étape, le solvant est éliminé par distillation sous vide et on obtient le produit brut de réaction ayant l'aspect d'une huile.

Conformément à une modalité très préférée de mise en oeuvre de la présente invention, le procédé précité s'applique bien, au départ de métaaminophénol, à la préparation des composés comprenant des mélanges à base de :
N-[(méth)allyloxy-3 phényl]maléimide + N-[(méth)allyloxy-3 (méth)allyl-4 phényl]maléimide + N-[(méth)-allyloxy-3 (méth)allyl-6 phényl]maléimide + éventuellement N-[(méth)allyloxy-3 di(méth)allyl-4,6 phényl]-maléimide.

A noter qu'au départ d'orthoaminophénol, on est conduit à des composés comprenant des mélanges à base de :
N-[(méth)allyloxy-2 phényl]maléimide + N-[(méth)allyloxy-2 (méth)allyl-3 phényl]maléimide + N-[(méth)-allyloxy-2 (méth)allyl-5 phényl]maléimide + éventuellement N-[(méth)allyloxy-2 di(méth)allyl -3,5 phényl]-maléimide. A noter encore qu'au départ de paraminophénol, on est conduit à des composés comprenant des mélanges à base de :
N-[(méth)allyloxy-4 phényl]maléimide + N-[(méth)allyloxy-4 (méth)allyl-3 phényl maléimide + éventuellement N-[(méth)allyloxy-4 di(méth)allyl-3,5 phényl]maléimide.

Les nouveaux composés comprenant des mélanges de monomaléimides de formules générales (I), (II) + éventuellement (III), lorsqu'ils sont utilisés avec un ou plusieurs bis-maléimides pour préparer des compositions thermodurcissables pour moulage ou pour imprégnation, apportent à ces compositions des propriétés mécaniques améliorées par rapport à des compositions préparés sans ces nouveaux monomaléimides.

Plus précisément, un autre objet de la présente invention consiste en de nouvelles compositions thermodurcissables, caractérisées en ce qu'elles comprennent le produit de réaction, à une température allant de 50 à 300 °C, entre les composés (a), (b), éventuellement (c), et (d) suivants :

(a) est un bis-imide ou une association de plusieurs bis-imides de formule générale (IV) :

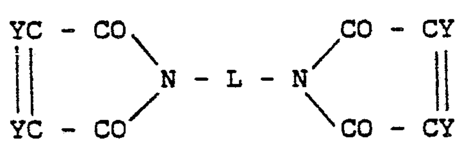

(IV)

dans laquelle:

-Y représente un atome d'hydrogène ou un groupement méthyle ;

-L représente un radical divalent tel qu'un radical cyclohexylène ; un radical phénylène ; le radical méthyl-4 phénylène-1,3 ; le radical méthyl-2 phénylène-1,3 ; le radical méthyl-5 phénylène-1,3 ; le radical diéthyl-2,5 méthyl-3 phénylène-1,4 et les radicaux de formule (V) :

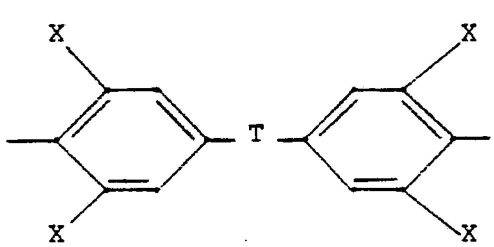

(V)

dans laquelle :

. T représente un lien valentiel simple ou un atome ou groupement suivant :

$-CH_2-$ ; 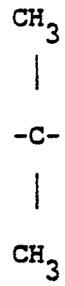 ; -O- ; 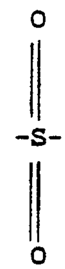 ;

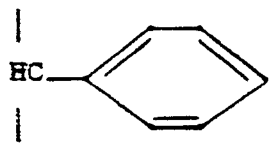 ; 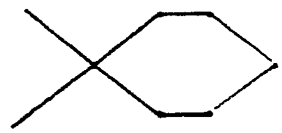

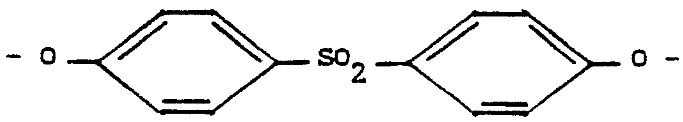

.X représente un atome d'hydrogène, un radical méthyle, éthyle ou isopropyle ;

(b) est le composé comprenant un mélange de N-(méth)allyloxyphénylmaléimide de formule (I) avec un ou plusieurs dérivés de substitution (méth)allylés de formules (II) et éventuellement (III), défini ci-avant dans le présent mémoire ;

(c), facultatif, est un composé organosilicique comportant dans sa molécule au moins un groupement hydroxyle lié à un atome de silicium ; et

(d) est un composé imidazole.

Le bis-maléimide de formule (IV) peut être choisi par exemple parmi :

- le N,N'-métaphénylène-bis-maléimide,
- le N,N'-paraphénylène-bis-maléimide,
- le N,N'-4,4'-diphénylméthane-bis-maléimide,
- le N,N'-4,4'-diphényléther-bis-maléimide,
- le N,N'-4,4'-diphénylsulfone-bis-maléimide,
- le N,N'-1,4-cyclohexylène-bis-maléimide,
- le N,N'-4,4'-(diphényl-1,1 cyclohexylidène)bis-maléimide,
- le N,N'-4,4'-(diphényl-2,2 propane)bis-maléimide,
- le N,N'-4,4'-triphénylméthane-bis-maléimide,
- le N,N'-1,3-méthyl-4 phénylène-bis-maléimide,
- le N,N'-1,3-méthyl-2 phénylène-bis-maléimide.

Parmi ces bis-maléimides on préfère plus particulièrement utiliser le N,N'-4,4'-diphénylméthane bis-maléimide, le N,N'-1,3-méthyl-4 phénylène bis-maléimide, le N,N'-1,3-méthyl-2 phénylène bis-maléimide et leurs mélanges.

Ces bis-maléimides peuvent être préparés selon les procédés décrits dans le brevet américain No. 3.018.290 et le brevet britannique No. 1.137.290.

Les composés organosiliciques hydroxylés qui entrent à titre facultatif dans le cadre de l'invention sont des composés connus répondant à la formule générale (VI) suivante :

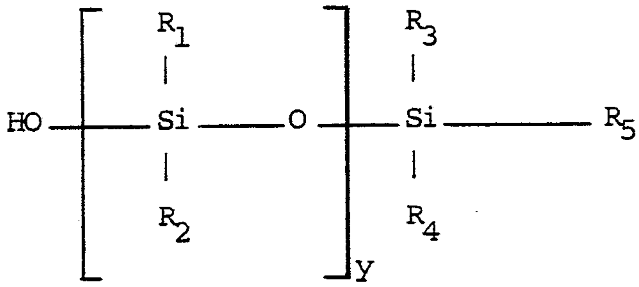

(VI)

dans laquelle $R_1$, $R_2$, $R_3$, $R_4$ et $R_5$, identiques ou différents, représentent :

- un groupement hydroxyle ou un groupement du type $-OR_6$ dans laquelle $R_6$ peut être un radical alkyle, linéaire ou ramifié, ayant de 1 à 6 atomes de carbone ou un radical phényle ;
- un atome d'hydrogène ;
- un radical alkyle, linéaire ou ramifié, ayant de 1 à 6 atomes de carbone et pouvant être éventuellement substitué par un ou plusieurs atomes de chlore ou de fluor ou par un groupement -CN ;
- un radical alcényle, linéaire ou ramifié, ayant de 2 à 6 atomes de carbone ;
- un radical phényle, éventuellement substitué par un ou plusieurs radicaux alkyles et'ou alcoxyles ayant de 1 à 4 atomes de carbone, ou par un ou plusieurs atomes de chlore ; et y est un nombre entier ou fractionnaire, de 0 à 1000.

Pour un composé organosilicique défini de formule (VI), y est en réalité toujours un nombre entier, mais comme il s'agit en l'occurrence de composés à structure polymérique (lorsque y est supérieur à 1) , on a rarement un seul composé, mais le plus souvent un mélange de composés de même structure chimique, qui diffèrent par le nombre d'unités récurrentes de leur molécule ; cela conduit à une valeur moyenne de y, qui peut être entière ou fractionnaire.

On peut caractériser les composés organosiliciques hydroxylés du type précité par le rapport du poids des groupements hydroxyle qu'ils possèdent au poids total de leur molécule.

La mise en oeuvre d'un composé organosilicique hydroxylé est une mesure permettant notamment de faciliter, lors de la préparation des compositions thermodurcissables selon la présente invention, le passage à l'état fondu des composés à groupements maléimides et de donner aussi à la résine thermodurcissable obtenue une plus grande fluidité à l'état fondu.

Quand on choisit d'utiliser des composés organosiliciques, les composés auxquels on fait appel de préférence pour exécuter la présente invention, sont les composés précités pour lesquels le rapport pondéral des groupements hydroxyle dans la molécule est au moins égal à 0,05 % et de préférence à 0,1 %.

Parmi les composés organosiliciques appartenant à ce groupe préféré, ceux qui conviennent tout particulièrement bien sont les composés de formule (VI) dans laquelle :

- $R_1$, $R_2$, $R_3$ et $R_4$, identiques ou différents, représentent un radical alkyle, linéaire ou ramifié, ayant de 1 à 6 atomes de carbone ou un radical alcényle, linéaire ou ramifié, ayant de 2 à 6 atomes de carbone ou un radical phényle ;
- $R_5$ représente un groupement hydroxyle ;
- y est un nombre, entier ou fractionnaire, de 0 à 250.

Il s'agit donc de silane-diols lorsque y est égal à 0 ou bien de polysiloxane-diols lorsque y est différent de 0.

Pour leur préparation on peut se reporter à l'ouvrage de W.NOLL "Chemistry and Technology of Silicones" (traduction anglaise de l'Edition allemande de 1968) édité par Academic Press de New-York.

Les composés organosiliciques qui conviennent tout particulièrement bien sont choisis dans le groupe constitué par :

- le diéthylsilane-diol
- le diphénylsilane-diol,
- le méthylphénylsilane-diol
- le tétraméthyl-1,1,3,3 disiloxane-diol-1,3
- le diméthyl-1,1 diphényl-3,3 disiloxane-diol-1,3
- le diméthyl-1,3 diphényl-1,3 disiloxane-diol-1,3
- l'hexaméthyl-1,1,3,3,5,5 trisiloxane-diol-1,5
- l'octaméthyl-1,1,3,3,5,5,7,7 tétrasiloxane-diol-1,7
- le décaméthyl-1,1,3,3,5,5,7,7,9,9 pentasiloxane-diol-1,9
- le dodécaméthyl-1,1,3,3,5,5,7,7,9,9,11,11 hexasiloxane-diol-1,11

- le pentaméthyl-1,3,5,7,9 pentaphényl-1,3,5,7,9 pentasiloxane-diol-1,9 ainsi que leurs homologues supérieurs correspondants.

Les composés organosiliciques hydroxylés convenant tout particulièrement bien peuvent également être des mélanges de deux ou plusieurs des composés précités. C'est ainsi que l'on peut utiliser par commodité des huiles ou résines polysiloxanes hydroxylées du commerce. Ce sont en particulier des huiles polyméthylpolysiloxanes-$\alpha,\omega$-dihydroxylées ayant de 0,2 à 0,3 % en poids de groupements hydroxyle (huile de Rhône-Poulenc 48 V 500), ou 10 à 12 % en poids de groupements hydroxyle (huile de Rhône-Poulenc 48 V 50) ou des huiles ou résines méthylphénylpolysiloxanes-$\alpha,\omega$-dihydroxylées ayant 4,5 % à 5 % en poids de groupements hydroxyle (huile 50606 de Rhône-Poulenc) ou de 7,5 à 8,5 % en poids de groupements hydroxyle (résine 50305 de Rhône-Poulenc) ; ces huiles ou résines du commerce sont données à titre d'exemple, mais il en existe d'autres pouvant convenir tout aussi bien.

Dans les compositons selon l'invention préparées à partir d'un ou plusieurs bis-imides de formule (IV) et du composé (b), on choisit les quantités de réactifs de manière à avoir, en poids par rapport au poids total de ces différents constituants :

-de 50 à 95 % de bis-imide(s),
-et de 5 à 50 % de composé (b).

Dans les compositions selon l'invention, préparées à partir d'un ou de plusieurs bis-imides de formule (II), du composé (b) et d'un composé organosilicique hydroxylé de formule (IV), on choisit les quantités de réactifs de manière à avoir, en poids par rapport au poids total de ces différents constituants :

- de 40 à 90 % de bis-imide(s),
- de 5 à 40 % de composé (b),
- et de 5 à 40 % de composé organosilicique hydroxylé.

Pour obtenir des compositions selon l'invention de ce type présentant des propriétés en flexion à chaud plus élevées, il est préférable de mettre en oeuvre une proportion de composé organosilicique représentant 5 à 20 % en poids du poids total de bis-imide(s), composé (b) et composé organosilicique hydroxylé.

Le composé imidazole (d) répond à la formule générale (VII) :

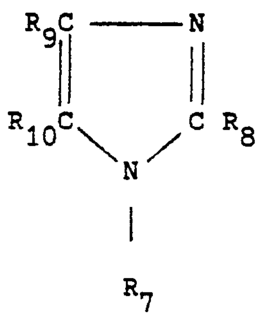

(VII)

dans laquelle $R_7$, $R_8$, $R_9$ et $R_{10}$, identiques ou différents, représentent : un atome d'hydrogène, un radical alkyle ou alcoxy ayant de 1 à 20 atomes de carbone, un radical vinyle, un radical phényle, un groupement nitro, $R_9$ pouvant former avec $R_{10}$ et les atomes de carbone auxquels sont liés ces radicaux un cycle unique comme par exemple un cycle benzénique, $R_7$ pouvant également représenter un groupement carbonyle lié à un deuxième cyle imidazole.

Comme exemples spécifiques de composés imidazole, on peut citer, en particulier, l'imidazole ou glyoxaline, le méthyl-1 imidazole, le méthyl-2 imidazole, le diméthyl-1,2 imidazole, le vinyl-1 imidazole, le vinyl-1 méthyl-2 imidazole, le benzimidazole, le carbonyldiimidazole.

Le composé imidazole est utilisé en quantités catalytiques. Selon la nature du composé imidazole et selon la vitesse de polymérisation souhaitée au stade de la mise en oeuvre, on utilise le composé imidazole à un taux compris entre 0,01 et 1 % en poids par rapport à l'ensemble des réactifs (a) + (b) + éventuellement (c).

De préférence on utilise un taux d'imidazole de 0,02 à 0,5 % en poids par rapport à la même référence.

Les compositions selon l'invention peuvent également contenir une N,N',N''-tris(hydroxyalkyl)-hexahydrotriazine ; on utilise plus particulièrement la N,N',N''-tris(hydroxyéthyl)hexahydrotriazine-1,3,5, la N,N',N''-tris(hydroxypropyl)hexahydrotriazine-1,3,5 et la N,N',N''-tris(hydroxybutyl)hexahydrotriazine-1,3,5.

On emploiera préférentiellement la N,N',N''-tris(hydroxyéthyl)hexahydrotriazine-1,3,5 disponible dans le commerce.

Lorsqu'elle est présente la N,N',N"-tris(hydroxyalkyl)hexahydrotriazine confère aux compositions thermodurcissables une pégosité plus élevée ainsi qu'une amélioration des propriétés thermomécaniques, notamment de la résistance à la flexion.

On utilise habituellement de 0 à 5 % en poids de N,N',N"-tris(hydroxyalkyl)hexahydrotriazine par rapport à l'ensemble des réactifs (a) + (b) + éventuellement (c).

Pour obtenir une bonne efficacité, il est préférable d'utiliser de 0,5 à 2 % en poids de N,N',N"-tris-(hydroxyalkyl)hexahydrotriazine par rapport à la même référence.

Les compositions selon l'invention conduisent après cuisson à des propriétés mécaniques, notamment en flexion, à température ambiante et à chaud (250 °C généralement) plus élevées que celles que l'on obtient avec des compositions antérieures, telles que celles décrites dans la demande de brevet français 83/17 218 publiée sous le numéro 2 553 780.

Divers adjuvants peuvent être incorporés dans les compositions selon l'invention. Ces adjuvants habituellement utilisés et bien connus de l'homme de l'art peuvent être par exemple des stabilisants ou des inhibiteurs de dégradation, des lubrifiants ou des agents de démoulage, des colorants ou des pigments, des charges pulvérulentes ou en particules comme des silicates, des carbonates, le kaolin, la craie, le quartz pulvérisé, le mica ou des microbilles de verre, etc ... On peut aussi incorporer des adjuvants modifiant la structure physique du produit obtenu comme par exemple des agents porogènes ou des renforçants fibreux : fibrilles de carbone, de polyimide, de polyamides aromatiques, whiskers, etc ....

Le procédé de fabrication est tel que la résine thermodurcissable prête à l'emploi présente suffisamment de souplesse et de collant en couche mince. En outre pour obtenir un matériau homogène après stratification, les réactions génératrices de composés très volatils aux températures de cuisson doivent être peu importantes. Dans ce but, lorsque les réacitifs de départ comprennent un silanediol, il est souhaitable de réaliser d'abord la plus grande partie de la réaction d'oligomérisation donnant de l'eau comme sous-produit ; cette eau peut être éliminée plus facilement en cours de fabrication de la résine.

Selon un premier procédé, on réalise un mélange des composés à groupements maléimides (a) et (b) et éventuellement du composé organosilicique hydroxylé (c) qui est fondu en absence de catalyseur à une température au plus égale à la température de fusion du maléimide le plus difficile à liquéfier, et généralement comprise entre 50 et 300 °C. Si le mélange réactionnel comprend un composé organosilicique riche en groupements hydroxyles, le mélange est dans ce cas maintenu fondu de façon à réaliser une partie de l'oligomérisation du silanediol ; de préférence on chauffera ce composé à environ 150 °C jusqu'à ce que 40 % environ des groupements hydroxyles initiaux aient disparu lors de l'oligomérisation de ce composé. On pourra, à titre de variante, réaliser cette oligomérisation avant introduction des composés à groupements maléimides.

Le composé imidazole (d) et le cas échéant la N,N',N"-tris(hydroxyalkyl)hexahydrotriazine sont ajoutés ensuite au mélange bien agité, de façon à permettre leur dispersion rapide.

Lorsque le catalyseur est particulièrement actif, il est souhaitable, afin d'éviter son encapsulation dans le réseau polymérique qu'il engendre, de l'ajouter avec un solvant compatible avec le milieu réactionnel. On peut ainsi utiliser un solvant tel que le triallylisocyanurate, le phtalate de diallyle ou le benzoate d'allyle.

On peut également employer un solvant volatil qui sera ultérieurement éliminé par vaporisaton sous pression réduite. En effet le mélange est dégazé afin d'éliminer les produits volatils qui sont contre-indiqués pour la préparation des stratifiés. Le mélange est coulé immédiatement après l'homogénéisation.

Selon un second procédé, applicable essentiellement en absence du composé organosilicique hydroxylé (c), les compositions selon l'invention peuvent être obtenues par chauffage direct des composés à groupements maléimides (a) et (b) et du composé imidazole (d), et le cas échéant de la N,N',N"-tris-(hydroxyalkyl)hexahydrotriazine, éventuellement dissous dans un solvant, à une température allant de 50 °C à 300 °C, jusqu'à l'obtention d'un mélange liquide homogène. Ensuite on dégaze et on coule rapidement la résine après homogénéisation. On peut, à titre de variante, mettre en oeuvre ce procédé en réalisant d'abord un mélange intime du composé (b) et du composé imidazole (d), et le cas échéant de la N,N',N"-tris(hydroxyalkyl)-hexahydrotriazine, éventuellement dissous dans un solvant, puis en ajoutant le composé (a) dans le mélange précité qui est chauffé à une température allant de 50 °C à 300 °C jusqu'à l'obtention d'un mélange liquide homogène.

Les compositions thermodurcissables selon l'invention possèdent une pégosité suffisante pour les applications telles que les stratifiés et les matériaux composites.

Les compositions peuvent être mises en oeuvre par des opérations de moulage ou d'imprégnation. Elles peuvent être utilisées pour la réalisation de revêtements, de collages, de stratifiés et matériaux composites renforcés. Le matériau de renforcement peut être sous forme de nappes tissées ou non tissées, d'éléments unidirectionnels ou de fibres coupées naturelles ou synthétiques telles que les filaments ou fibres de verre, de bore, de carbone, de tungstène, de silicium, de polyamide-imides ou polyamides

aromatiques. Les compositions présentent un intérêt tout particulier pour l'obtention d'articles intermédiaires préimprégnés sans solvant. L'imprégnation du matériau fibreux peut être effectuée par application des techniques usuelles telles que l'immersion, l'enduction au racle ou au rideau ou l'imprégnation par transfert. Le film transférable et les articles préimprégnés peuvent être utilisés directement ou bien être emmagasinés en vue d'un emploi ultérieur ; ils conservent de manière très satisfaisantes leurs propriétés au cours d'un stockage au froid entre 0 et 10°C.

Les matériaux imprégnés sont utilisables pour la réalisation de pièces ayant des formes et des fonctions variées dans de nombreuses industries comme par exemple dans l'aéronautique. Ces pièces qui peuvent être des pièces de révolution sont obtenues par placage de plusieurs couches de préimprégnés sur une forme ou un support.

On effectue ensuite la réticulation dans les conditions technologiques habituelles relatives aux matériaux composites et en particulier à des températures comprises entre 100 et 300°C.

On peut utiliser aussi les préimprégnés comme renforts ou moyens de réparation de pièces détériorées.

Mais il est aussi possible de concevoir des pièces selon les techniques de l'enroulement filamentaire avec ou sans support, ou de l'injection moulage ou de la pultrusion.

On peut ainsi obtenir des produits conformés à haute résistance mécanique et thermique.

Les exemples suivants sont donnés à titre illustratif de l'invention :

EXEMPLE 1

Procédé de préparation d'un composé selon l'invention comprenant un mélange à base des constituants suivants : N-(allyloxy-3 phényl)maléimide + N-(allyloxy-3 allyl-4 phényl)maléimide + N-(allyloxy-3 allyl-6 phényl)maléimide + N-(allyloxy-3 diallyl-4,6 phényl)maléimide.

1. Première étape :

Dans un reacteur en verre muni d'une agitation centrale, d'un réfrigérant ascendant, d'un électrode de mesure de pH, d'une sonde de mesure de température et d'un système de chauffage ou de refroidissement, on charge :

-186 g d'anhydride maléique,

-et 540 cm$^3$ de méthylisobutylcétone,

et on chauffe à 40° C.

On prépare une solution de 196 g de métaaminophénol dans 1,2 litres de méthylisobutylcétone et on coule cette solution en 1 heure ; pendant ce temps, la température monte de 40° C à 60° C. Après la coulée, on laisse réagir encore à 60° C pendant 20 minutes.

On obtient à la fin de cette étape l'acide N-(hydroxy-3 phényl)maléamique. Le rendement est de 100 % par rapport à l'aminophénol de départ.

2. Deuxième étape :

On ajoute dans le milieu réactionnel obtenu à l'issue de la première étape 720 cm$^3$ d'eau et 240 cm$^3$ de soude aqueuse à 30 % en poids et on ajuste alors le pH du milieu à une valeur de 10,5 avec la soude aqueuse précitée. La température est amenée ensuite à 70° C et on coule 413 g de chlorure d'allyle en 4 heures. La température s'élève pendant ce temps à 80° C. Après coulée, on maintient cette température pendant encore 2 autres heures. Pendant la coulée du chlorure d'allyle, le pH a été maintenu à la valeur de 10,5 par injection de la soude aqueuse à 30 %.

En fin d'étape on amène le pH à une valeur voisine de 1 par ajout d'une solution aqueuse d'acide sulfurique à 50 % en poids, puis on élimine la phase aqueuse par décantation et on obtient une masse réactionnelle organique de 2150 g.

Le rendement, par rapport à l'acide hydroxyphénylmaléamique est de :

-77 % en acide N-(allyloxy-3 phényl)maléamique,

-20 % en acides N-(allyloxy-3 allyl-4 phényl)maléamique et N-(allyloxy-3 allyl-6 phényl)maléamique,

-3 % en acide N-(allyloxy-3 diallyl-4,6 phényl)maléamique.

3.Troisième étape :

Dans le milieu final de la deuxième étape, on ajoute 3,2 $cm^3$ d'une solution aqueuse à 20 % en poids d'acétate de nickel et on distille par azéotropisme 72,5 $cm^3$ d'eau restant dans la phase organique.

On charge alors 204 g d'anhydride acétique et 51 g de triéthylamine. Le mélange réactionnel est chauffé à 65° C pendant 1 heure 30 minutes, puis il est refroidi à 20° C. On ajoute ensuite 800 $cm^3$ d'eau et on amène le pH à la valeur de 7 par addition de soude aqueuse à 30 %.

On élimine la phase aqueuse et la phase organique est lavée deux fois avec 100 $cm^3$ d'eau. Le solvant de la phase organique est distillé sous pression réduite jusqu'à une valeur de $1,33.10^2$ Pa vers 50-60° C. On obtient ainsi 250 g d'une huile visqueuse constituant le produit brut de réaction.

Le rendement molaire global, par rapport à l'aminophénol de départ, est de :

-40 % en N-(allyloxy-3 phényl)maléimide,

-10 % en N-(allyloxy-3 allyl-4 phényl)maléimide et N-(allyloxy-3 allyl-6 phényl)maléimide,

-1,5 % en N-(allyloxy-3 diallyl-4,6 phényl)maléimide.

Le produit brut de réaction est un composé comprenant un mélange formé de :

-74,5 % en poids de N-(allyloxy-3 phényl)maléimide,.

-21,8 % en poids de N-(allyloxy-3 allyl-4 phényl)maléimide et de N-(allyloxy-3 allyl-6 phényl)maléimide,

-et 3,7 en poids de N-(allyloxy-3 diallyl-4,6 phényl)maléimide.

EXEMPLE 2

Dans un réacteur en verre muni d'une tubulure latérale et d'un agitateur de type ancre , on introduit 20 g du produit brut issu du procédé décrit ci-avant dans l'exemple 1. On plonge le réacteur dans un bain d'huile préalablement chauffé à 160° C. Après 2 minutes d'homogénéisation, on ajoute sous agitation à 160° C, un mélange de poudres composé de 68 g de N,N-4,4'-diphénylméthane-bis-maléimide et de 10 g de diphénylsilanediol, en 4 minutes.

La masse réactionnelle devient limpide en 7 minutes et on baisse la température à 150° C et on laisse agir 7 autres minutes, puis l'on refroidit à 130° C en 15 minutes. On applique alors durant 4 minutes une pression réduite d'environ 130 Pa.

On rétablit ensuite la pression atmosphérique dans le réacteur et on introduit alors la solution catalytique : 0,1 g d'imidazole dans 1,9 g de triallylisocyanurate en 1 minute, puis on laisse homogénéiser 4 minutes et on applique pendant 5 minutes une pression réduite d'environ 130 Pa.

On coule finalement la masse réactionnelle dans un moule préchauffé à 120° C. On peut ainsi préparer des plaques de dimensions 140 $\times$ 100 $\times$ 4 mm qui vont subir le cycle de cuisson suivant :

- 60 min entre 120° C et 150° C,
- 60 min à 150° C,
- 40 min entre 150° C et 200° C,
- 120 min à 200° C,
- 40 min entre 200° C et 250° C,
- 16 h à 250° C,
- et 2 h entre 250° C et 25° C.

Les plaques de résine recuite ainsi obtenues sont opaques et sans défauts. Elles sont alors découpées pour obtenir des éprouvettes de dimension 30 $\times$ 7 $\times$ 4 mm, sur lesquelles on mesure la résistance à la rupture et le module d'Young en flexion trois points (distance entre appuis 25,4 mm ; Machine INSTRON).

Les valeurs des propriétés mécaniques sont reportées dans le tableau suivant :

. Temps 0 :

| Température | Rf(MPa) | Mf(MPa) |
|---|---|---|
| 25° C | 123 | 2 700 |
| 250° C | 64,7 | 2 200 |

. après 1000 h à 250° C :

| Température | Rf(MPa) | Mf(MPa) |
|---|---|---|
| 25°C | 102,3 | 3 000 |
| 250° C | 54,4 | 2 420 |

EXEMPLE 3 :

Dans le réacteur décrit ci-avant à l'exemple 2, on introduit à température ambiante : 19, 36 g du produit brut issu du procédé décrit ci-avant dans l'exemple 1 et 0,08 g d'imidazole.

On plonge le réacteur dans un bain d'huile préchauffé à 160° C. On agite durant 2 minutes pour homogénéiser le catalyseur. On additionne alors 60,64 g de N,N'-4,4'-diphénylméthane-bis-maléimide en 5 minutes sous agitation. Deux minutes après la fin de l'addition, la masse réactionnelle est homogène. On laisse réagir encore 5 minutes et on applique une pression réduite de 130 Pa pendant 2 minutes.

On coule ensuite la masse réactionnelle dans un moule préchauffé à 150° C. Le prépolymère obtenu est ainsi moulé, puis durci et testé comme indiqué ci-avant dans l'exemple 2. A noter que le cycle de cuisson est ici suivant :
- 1 h à 150° C,
- 50 min de 150° C à 200° C,
- 2 h à 200° C,
- 50 min de 200° C à 250° C,
- 16 h à 250° C,
- et 2 h de 250° C à 25° C.

Résultats de flexion des éprouvettes de polymère durci :
- à 25° C : Rf = 134 MPa
  Mf = 3100 MPa
- à 250° C : Rf = 90,6 MPa
  Mf = 2700 MPa

EXEMPLE 4

On opère comme indiqué dans l'exemple 3 avec les charges suivantes :
- 75,8 g de N,N'-4,4'-diphénylméthane-bis-maléimide,
- 24,2 g du produit brut issu du procédé de l'exemple 1,
- 0,1 g d'imidazole.

La masse réactionnelle est seulement coulée sur plateaux froids 15 minutes après la fin d'addition du bis-maléimide.

Le prépolymère est broyé finement pour donner une poudre jaune dont le point de ramolissement est égal à 77,6° C. Ce prépolymère est soluble à 50 % (P/P) dans la cyclohexanone et le collodion obtenu est stable pendant au moins 5 jours ; les caractéristiques du collodion obtenu sont les suivantes :
-Temps de gel à 150° C = 42,6 min.
-Viscosité dynamique à 25° C : Temps zéro = 1,64 poises, après 24 h = 2,1 poises.

A partir de ce collodion, il est possible par enduction de réaliser des stratifiés fibre de verre (tissu 7628 de la Société PORCHER ayant un grammage de 200 g/m$^2$ ; ce tissu a subi un traitement par le gamma-aminopropyltriéthoxysilane A 1100 d'UNION CARBIDE) de 6 plis (dimmension 145 × 100 mm)

Après séchage de 5 minutes à 150° C, on applique sur chacune des faces du stratifié, un feuillard de

cuivre de 35 micromètres d'épaisseur et l'on respecte le cycle de cuisson suivant :
- 15 min à 160° C + 1h15 à 180° C sous 40 bars
- et 4 h à 235° C.

La force d'arrachement du cuivre mesurée sur machine INSTRON par traction du cuivre à 90° d'angle est de l'ordre de 14,7 N/cm.

**Revendications**

1°) Composés maléimides caractérisés en ce qu'ils comprennent :
- un mélange d'un monomère de formule :

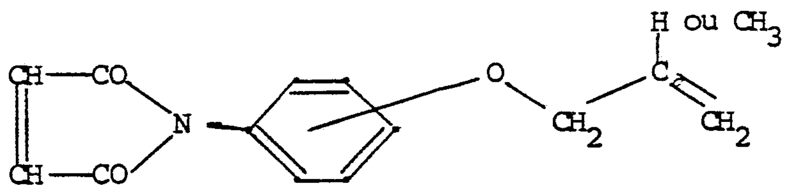

(I)

dans laquelle le radical allyloxy ou méthallyloxy est en position ortho, méta ou para par rapport à l'atome de carbone du cycle benzénique relié à l'azote.
- avec :

. au moins un dérivé monosubstitué de formule :

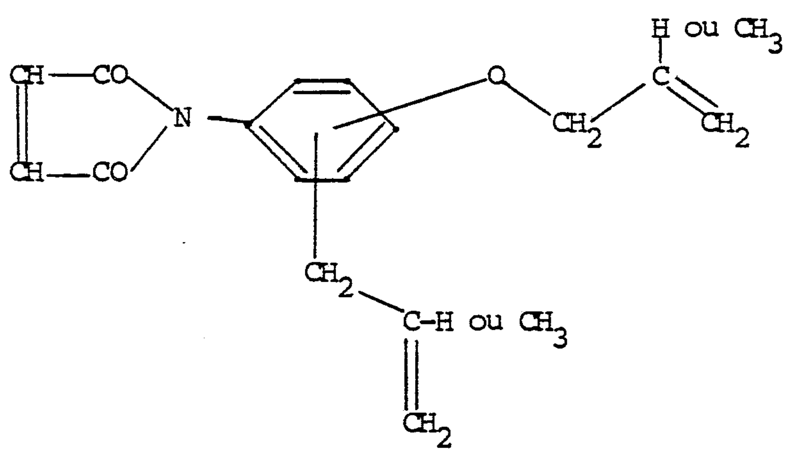

(II)

. et éventuellement un ou plusieurs dérivés disubstitués de formule :

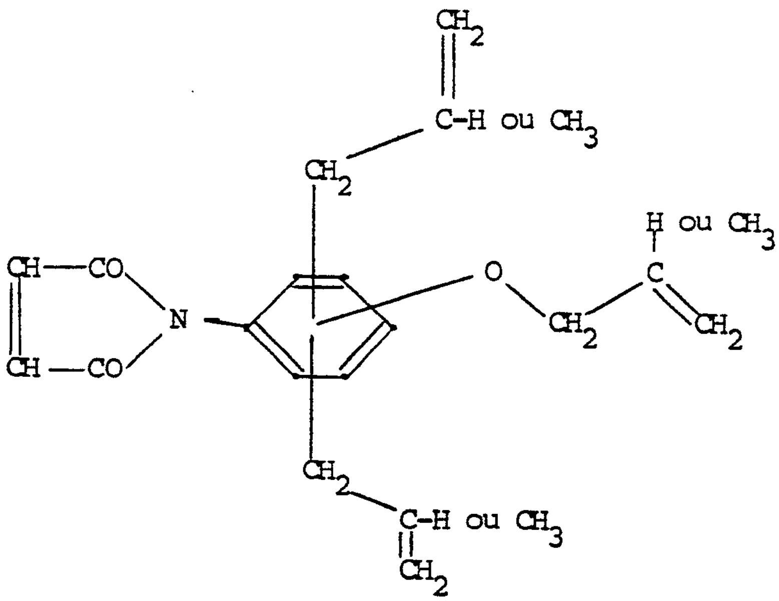

(III)

2°) Composés selon la revendication 1, caractérisés en ce qu'il comprennent un mélange qui renferme en poids :

- au moins 30 %, et de préférence de 50 % à 80 % de N-(méth)allyloxyphénylmaléimide de formule (I),
- de 5 % à 50 % et de préférence de 10 % à 35 % de dérivé(s) mono-(méth)allyl substitué(s) de formule (II),

-et de 0 % à 20 % et de préférence de 0 % à 15 % de dérivé(s) di(méth)allyl substitué(s) de formule (III),

3°) Procédé de préparation des composés selon l'une quelconque des revendications 1 et 2, caractérisé en ce qu'il consiste à réaliser les 3 étapes suivantes qui ont enchaînées dans le même réacteur :

-la première étape consiste à faire réagir en milieu solvant un aminophénol avec de l'anhydride maléique en opérant à une température allant de 20° C à 200° C, pendant une durée allant, selon la température choisie, de 30 minutes à 2 heures et elle conduit à un premier milieu réactionnel comprenant un acide N-(hydroxylphényl)maléamique ;

-la deuxième étape consiste à réaliser une réaction de (méth)allylation de l'acide précité en faisant réagir le premier milieu réactionnel précité avec un halogénure de (méth)allyle en opérant à un pH qui doit être ajusté et maintenu à une valeur constante comprise entre 7 et 14 par addition d'une quantité déterminée d'une solution aqueuse alcaline et à une température allant de 40 °C à 150 °C et elle conduit, après acidification et élimination de la phase aqueuse, à un second milieu réactionnel organique comprenant un acide N-[(méth(allyloxyphényl]maléamique, et éventuellement un ou plusieurs acides N-[(méth)-allyloxy,di(méth)allylphényl]maléamiques ;

-la troisième étape consiste à réaliser une réaction de cyclisation des acides maléamiques précités en faisant réagir le second milieu réactionnel précité avec un anhydride d'acide carboxylique inférieur, en présence d'une amine tertiaire et éventuellement d'un catalyseur, puis à éliminer le solvant de la réaction et elle conduit à un produit brut de réaction qui est un composé comprenant un mélange formé par un N-(méth)allyloxyphénylmaléimide, un ou plusieurs N-[(méth)allyloxy, (méth)allylphényl]maléimides et éventuellement un ou plusieurs N-[(méth)allyloxy,di(méth)allylphényl]maléimides.

4°) Nouvelles compositions thermodurcissables, caractérisées en ce qu'elles comprennent le produit de réaction, à une température allant de 50 °C à 300 °C, entre les composés (a), (b), éventuellement (c), et (d) suivants :

(a) est un bis-imide ou une association de plusieurs bis-imides de formule générale (IV) :

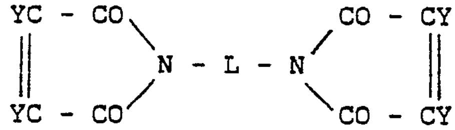

(IV)

dans laquelle :

-Y représente un atome d'hydrogène ou un groupement méthyle ;

-L représente un radical divalent tel qu'un radical cyclohexylène : un radical phénylène : le radical méthyl-4 phénylène-1,3 ; le radical méthyl-2 phénylène-1.3 ; le radical méthyl-5 phénylène-1.3 : le radical diéthyl-2,5 méthyl-3 phénylène-1,4 et les radicaux de formule (V) :

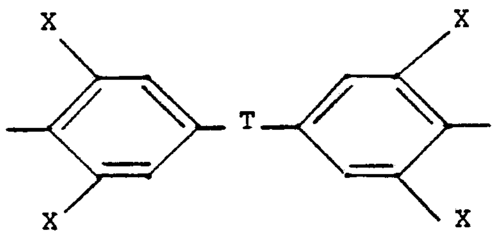

(V)

dans laquelle :

. T représente un lien valentiel simple ou un atome ou groupement suivant :

-$CH_2$- ; 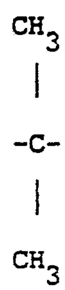 ; -O- ; 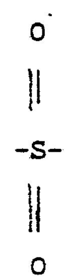 ;

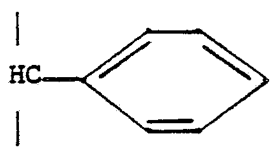 ; 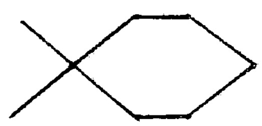

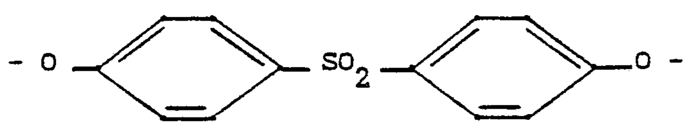

. X représente un atome d'hydrogène, un radical méthyl, éthyle ou isopropyle ;

(b) est le composé défini ci-avant dans l'une quelconque des revendications 1 et 2;

(c), facultatif, est un composé organosilicique comportant dans sa molécule au moins un groupement hydroxyle lié à un atome de silicium ; et

(d) est un composé imidazole.

5°) Compositions selon la revendication 4, caractérisées en ce que le bis-maléimide (a) de formule (IV) est choisi parmi :

- le N,N'-métaphénylène-bis-maléimide,
- le N,N'-paraphénylène-bis-maléimide,
- le N,N'-4,4'-diphénylméthane-bis-maléimide,
- le N,N'-4,4'-diphényléther-bis-maléimide,
- le N,N'-4,4'-diphénylsulfone-bis-maléimide,
- le N,N'-1,4-cyclohexylène-bis-maléimide,
- le N,N'-4,4'-(diphényl-1,1 cyclohexylidène)bis-maléimide,
- le N,N'-4,4'-(diphényl-2,2 propane)bis-maléimide,
- le N,N'-4,4'-triphénylméthane-bis-maléimide,
- le N,N'-1,3-méthyl-4 phénylène-bis-maléimide,
- le N,N'-1,3-méthyl-2 phénylène-bis-maléimide.

6°) Compositions selon l'une des revendications 4 ou 5, caractérisées en ce que l'on utilise comme composé (b) le produit brut obtenu par mise en oeuvre du procédé défini ci-avant dans la revendication 3.

7°) Compositions selon l'une des revendications 4 à 6, caractérisées en ce que le composé organosilicique (c), quand on en utilise un, répond à la formule générale (VI) :

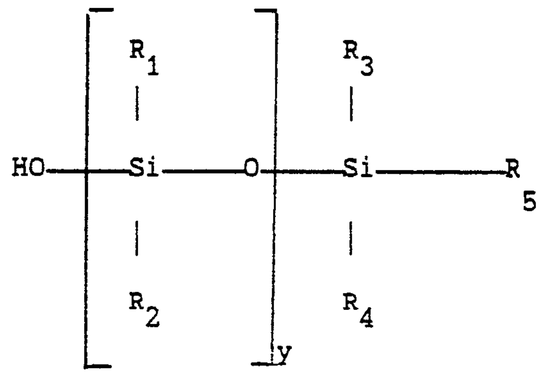

(VI)

dans laquelle $R_1$, $R_2$, $R_3$, $R_4$ et $R_5$, identiques ou différents, représentent :

- un groupement hydroxyle ou un groupement du type $-OR_6$ dans laquelle $R_6$ peut être un radical alkyle, linéaire ou ramifié, ayant de 1 à 6 atomes de carbone ou un radical phényle ;
- un atome d'hydrogène ;
- un radical alkyle, linéaire ou ramifié, ayant de 1 à 6 atomes de carbone et pouvant être éventuellement substitué par un ou plusieurs atomes de chlore ou de fluor ou par un groupement -CN ;
- un radical alcényle, linéaire ou ramifié, ayant de 2 à 6 atomes de carbone ;
- un radical phényle, éventuellement substitué par un ou plusieurs radicaux alkyles et/ou alcoxy ayant de 1 à 4 atomes de carbone, ou par un ou plusieurs atomes de chlore ; et y est un nombre entier ou fractionnaire, de 0 à 1 000.

8°) Compositions selon l'une des revendications 4 à 7, caractérisées en ce que le composé imidazole (d) répond à la formule générale (VII) :

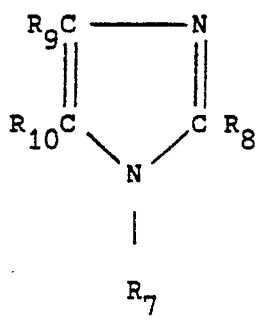

(VII)

dans laquelle $R_7$, $R_8$, $R_9$ et $R_{10}$, identiques ou différents, représentent : un atome d'hydrogène, un radical alkyle ou alcoxy ayant de 1 à 20 atomes de carbone, un radical vinyle, un radical phényle, un groupement nitro, $R_9$ pouvant former avec $R_{10}$ et les atomes de carbone auxquels sont liés ces radicaux un cycle unique comme par exemple un cycle benzénique, $R_7$ pouvant également représenter un groupement carbonyle lié à un deuxième cyle imidazole.

(9°) Compositions selon l'une des revendications 4 à 8, caractérisées en ce que le bis-maléimide (a) utilisé est choisi parmi le N,N'-4,4'-diphénylméthane-bis-maléimide, le N,N'-1,3-méthyl-4 phénylène-bis-maléimide, le N,N'-1,3-méthyl-2 phénylène-bis-maléimide et leurs mélanges.

10°) Compositions selon l'une des revendications 4 à 9, caractérisées en ce que le composé organosilicique hydroxylé (c) de formule (VI) qui peut être utilisé est le diphénylsilanediol.

11°) Compositions selon l'une des revendications 4 à 6 et 8 et 9, caractérisées en ce qu'elles sont obtenues à partir d'un ou plusieurs bis-imides (a) de formule (II) et d'un composé (b) avec des quantités de réactifs telles que l'on ait, en poids par rapport au poids total de ces réactifs :

- de 50 à 95 % de bis-imide(s) (a),
- et de 5 à 50 % de composé (b).

12°) Compositions selon l'une des revendications 4 à 10, caractérisées en ce qu'elles sont obtenues à partir d'un ou de plusieurs bis-imides (a) de formule (II), d'un composé (b) et d'un composé organosilicique hydroxylé (c) avec des quantités de réactifs telles que l'on ait, en poids par rapport au poids total de ces réactifs :
- de 40 à 90 % de bis-imide(s) (a),
- de 5 à 40 % de composé (b),
- et de 5 à 40 % de composé organosilicique hydroxylé (c).

13°) Compositions selon la revendication 12, caractérisées en ce qu'elles sont préparées à partir de 5 à 20 % en poids de composé organosilicique hydroxylé (c) par rapport au poids total de bis-imide(s) (a), de composé (b) et de composé organosilicique hydroxylé (c).

14°) Compositions selon l'une des revendications 4 à 13, caractérisées en ce qu'elles contiennent de 0,01 à 1 % et de préférence de 0,02 à 0,5 % en poids de composé imidazole par rapport à l'ensemble des réacitifs (a) + (b) + éventuellement (c).

15°) Compositions selon l'une des revendications 4 à 14, caractérisées en ce qu'elles contiennent de 0 à 5 % et de préférence de 0,5 à 2 % en poids de N,N',N''-tris(hydroxyalkyl)hexahydrotriazine par rapport à l'ensemble des réactifs (a) + (b) + éventuellement (c).

16°) Procédé de préparation de compositions selon l'une des revendications 4 à 15, caractérisé en ce que l'on réalise un mélange des composés à groupements maléimides (a) et (b) et éventuellement du composé organosilicique hydroxylé (c), qui est fondu à une température allant de 50 °C à 300 °C, puis que l'on ajoute le composé imidazole (d), et le cas échéant la N,N',N''-tris-(hydroxylalkyl)hexahydrotriazine, éventuellement dissous dans un solvant, sous forte agitation, puis que l'on dégaze et que l'on coule rapidement la résine immédiatement après homogénéisation.

17°) Procédé de préparation de compositions sans faire appel à un composé organosilicique hydroxylé (c) selon l'une des revendications 4 à 6, 8, 9, 11, 14 et 15, caracterisé en ce que l'on réalise d'abord un mélange intime du composé (b) et du composé imidazole (d), et le cas échéant de la N,N',N''-tris-(hydroxyalkyl)hexahydrotriazine, éventuellement dissous dans un solvant, puis que l'on ajoute le composé -(a) dans le mélange précité qui est chauffé à une température allant de 50 °C à 300 °C, puis que l'on dégaze et que l'on coule la résine immédiatement après homogénéisation.

18°) Procédé selon l'une des revendications 16 et 17, caractérisé en ce que le composé imidazole, et le cas échéant la N,N',N''-tris(hydroxyalkyl) hexahydrotriazine, sont dissous avant leur addition, dans du triallylisocyanurate, du phtalate de diallyle ou du benzoate d'allyle.

19°) Produits conformés préparés à partir des compositions selon l'une des revendications 4 à 15.

20°) A titre de produits industriels nouveaux :
- d'une part les N-[(méth)allyloxy,(méth)allylphényl] maléimides de formule (II) et les N-[(méth)allylloxy, di-(méth)allylphényl] maléimides de formule (III) définis ci-avant dans l'une quelconque des revendications 1 et 3 ;
- et d'autre part les composés intermédiaires obtenus en fin de deuxième étape du procédé selon la revendication 3 comprenant un mélange à base d'un acide N-[(méth)allyloxyphényl] maléamique, d'un ou plusieurs acides N-[(méth)allyloxy, di(méth)allylphényl] maléamiques, ainsi que les différents acides maléamiques pris en tant que tels entrant dans la constitution dudit mélange.

Revendications pour l'Etat contractant suivant : ES

1°) Procédé de préparation de composés maléimides comprenant :
- un mélange d'un monomère de formule :

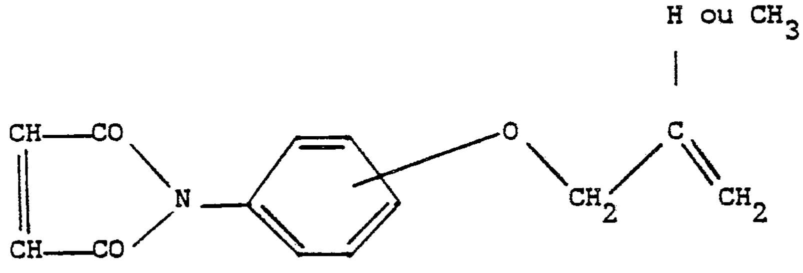

(I)

dans laquelle le radical allyloxy ou méthallyloxy est en position ortho, métha ou para par rapport à l'atome de carbone du cyle benzénique relié à l'azote.

- avec :

. au moins un dérivé monosubstitué de formule :

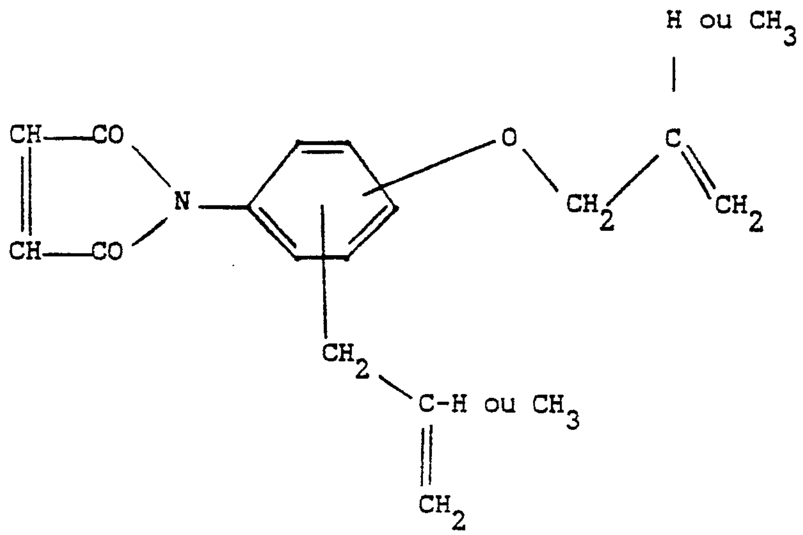

(II)

. et éventuellement un ou plusieurs dérivés disubstitués de formule :

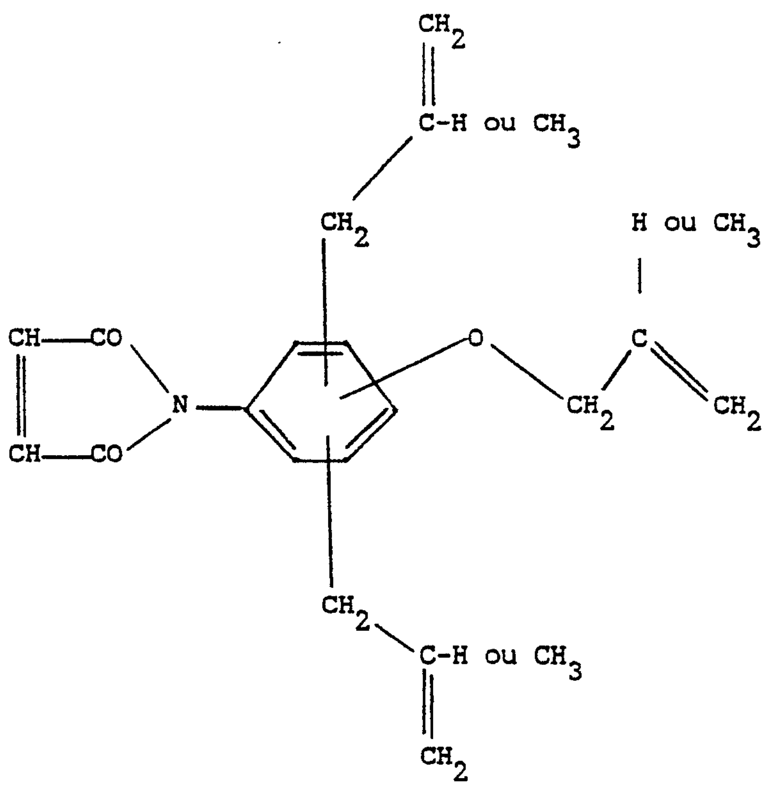

(III)

caractérisé en ce qu'il consiste à réaliser les trois étapes suivantes qui sont enchaînées dans le même réacteur :

-la première étape consiste à faire réagir en milieu solvant un aminophénol avec de l'anhydride maléique en opérant à une température allant de 20 °C à 200 °C, pendant une durée allant, selon la température choisie, de 30 minutes à 2 heures et elle conduit à un premier milieu réactionnel comprenant un acide N-(hydroxylphényl)maléamique ;

-la deuxième étape consiste à réaliser une réaction de (méth)allylation de l'acide précité en faisant réagir le premier milieu réactionnel précité avec un halogénure de (méth)allyle en opérant à un pH qui doit

être ajusté et maintenu à une valeur constante comprise entre 7 et 14 par addition d'une quantité déterminée d'une solution aqueuse alcaline et à une température allant de 40 °C à 150 °C et elle conduit. après acidification et élimination de la phase aqueuse, à un second milieu réactionnel organique comprenant un acide N-[(méth)allyloxyphényl] maléamique, un ou plusieurs acides N-[(méth)allyloxy,(méth)-allyphényl] maléamiques et éventuellement un ou plusieurs acides N-[(méth)allyloxy,di(méth)allylphényl] maléamiques ;

-la troisième étape consiste à réaliser une réaction de cyclisation des acides maléamiques précités en faisant réagir le second milieu réactionnel précité avec un anhydride d'acide carboxylique inférieur, en présence d'une amine tertiaire et éventuellement d'un catalyseur, puis à éliminer le solvant de la réaction et elle conduit à un produit brut de réaction qui est un composé comprenant un mélange formé par un N-(méth)allyloxyphénylmaléimide de formule (I), un ou plusieurs N-[(méth)allyloxy, (méth)allylphényl] maléimides de formule (II) et éventuellement un ou plusieurs N-[(méth)allyloxy,di(méth)allylphényl] maléimides de formule (III).

2°) Procédé de préparation de compositions thermodurcissables, caractérisé en ce que l'on fait réagir, à une température allant de 50 °C à 300 °C, les composés (a), (b), éventuellement (c), et (d) suivants :

(a) est un bis-imide ou une association de plusieurs bis-imides de formule générale (IV) :

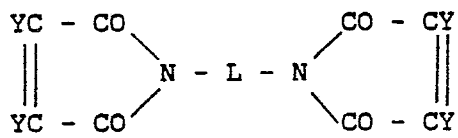

(IV)

dans laquelle :

-Y représente un atome d'hydrogène ou un groupement méthyle :

-L représente un radical divalent tel qu'un radical cyclohexylène ; un radical phénylène ; le radical méthyl-4 phénylène-1,3 ; le radical méthyl-2 phénylène-1,3 ; le radical méthyl-5 phénylène-1,3 ; le radical diéthyl-2,5 méthyl-3 phénylène-1,4 et les radicaux de formule (V) :

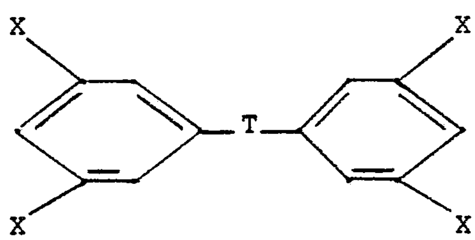

(V)

dans laquelle :

. T représente un lien valentiel simple ou un atome ou groupement suivant :

$-CH_2-$ ; 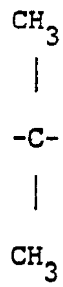 ; $-O-$ ; 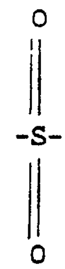 ;

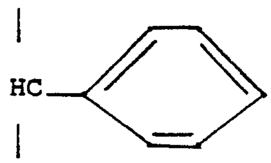 ; 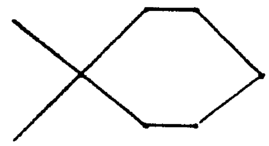

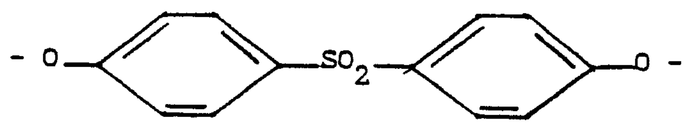

. X représente un atome d'hydrogène, un radical méthyle, éthyle ou isopropyle ;

(b) est l'un des composés maléimides obtenus ci-avant dans la revendication 1 ;

(c), facultatif, est un composé organosilicique comportant dans sa molécule au moins un groupement hydroxyle lié à un atome de silicium ; et

(d) est un composé imidazole.

3°) Procédé selon la revendication 2, caractérisé en ce que le bis-maléimide (a) de formule (IV) est choisi parmi :

- le N,N'-métaphénylène-bis-maléimide,
- le N,N'-paraphénylène-bis-maléimide,
- le N,N'-4,4'-diphénylméthane-bis-maléimide,
- le N,N'-4,4'-diphényléther-bis-maléimide,
- le N,N'-4,4'-diphénylsulfone-bis-maléimide,
- le N,N'-1,4-cyclohexylène-bis-maléimide,
- le N,N'-4,4'-(diphényl-1,1 cyclohxylidène)bis-maléimide,
- le N,N'-4,4'-(diphényl-2,2 propane)bis-maléimide,
- le N,N'-4,4'-triphénylméthane-bis-maléimide,
- le N,N'-1,3-méthyl-4 phénylène-bis-maléimide,
- le N,N'-1,3-méthyl-2 phénylène-bis-maléimide.

4°) Procédé selon l'une des revendications 2 ou 3, caractérisé en ce que le composé organosilicique (c), quand on en utilise un, répond à la formule générale (VI) :

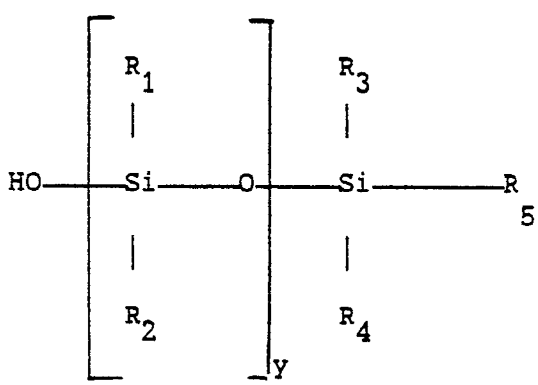 (VI)

dans laquelle $R_1$, $R_2$, $R_3$, $R_4$ et $R_5$, identiques ou différents, représentent :
- un groupement hydroxyle ou un groupement du type $-OR_6$ dans laquelle $R_6$ peut être un radical alkyle, linéaire ou ramifié, ayant de 1 à 6 atomes de carbone ou un radical phényle ;
- un atome d'hydrogène ;
- un radical alkyle, linéaire ou ramifié, ayant de 1 à 6 atomes de carbone et pouvant être éventuellement substitué par un ou plusieurs atomes de chlore ou de fluor ou par un groupement -CN ;
- un radical alcényle, linéaire ou ramifié, ayant de 2 à 6 atomes de carbone ;
- un radical phényle, éventuellement substitué par un ou plusieurs radicaux alkyles et ou alcoxy ayant de 1 à 4 atomes de carbone, ou par un ou plusieurs atomes de chlore ; et y est un nombre entier ou fractionnaire, de 0 à 1 000.

5°) Procédé selon l'une des revendications 2 à 4, caractérisé en ce que le composé imidazole (d) répond à la formule générale (VII) :

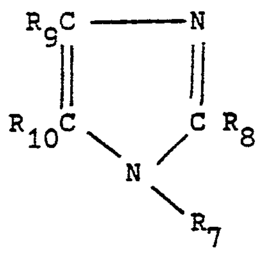

(VII)

dans laquelle $R_7$, $R_8$, $R_9$ et $R_{10}$, identiques ou différents, représentent : un atome d'hydrogène, un radical alkyle ou alcoxy ayant de 1 à 20 atomes de carbone, un radical vinyle, un radical phényle, un groupement nitro, $R_9$ pouvant former avec $R_{10}$ et les atomes de carbone auxquels sont liés ces radicaux un cycle unique comme par exemple un cycle benzénique, $R_7$ pouvant également représenter un groupement carbonyle lié à un deuxième cyle imidazole.

6°) Procédé selon l'une des revendications 2 à 5, caractérisé en ce que le bis-maléimide (a) utilisé est choisi parmi le N,N'-4,4'-diphénylméthane-bis-maléimide, le N,N'-1,3-méthyl-4 phénylène-bis-maléimide, le N,N'-1,3'-méthyl-2 phénylène-bis-maléimide et leurs mélanges.

7°) Procédé selon l'une des revendications 2 à 6, caractérisé en ce que le composé organosilicique hydroxylé (c) de formule (VI) qui peut être utilisé est le diphénylsilanediol.

8°) Procédé selon l'une des revendications 2, 3, 5 et 6, caractérisé en ce qu'il est mis en oeuvre à partir d'un ou plusieurs bis-imides (a) de formule (II) et d'un composé (b) avec des quantités de réactifs telles que l'on ait, en poids par rapport au poids total de ces réactifs :
- de 50 à 95 % de bis-imide(s) (a),
- et de 5 à 50 % de composé (b).

9°) Procédé selon l'une des revendications 2 à 7, caractérisé en ce qu'il est mis en oeuvre à partir d'un ou de plusieurs bis-imides (a) de formule (II), d'un composé (b) et d'un composé organosilicique hydroxylé (c) avec des quantités de réactifs telles que l'on ait, en poids par rapport au poids total de ces réactifs :
- de 40 à 90 % de bis-imide(s) (a),
- de 5 à 40 % de composé (b),
- et de 5 à 40 % de composé organosilicique hydroxylé (c).

10°) Procédé selon la revendication 9, caractérisé en ce qu'il est mis en oeuvre à partir de 5 à 20 % en poids de composé organosilicique hydroxylé (c) par rapport au poids total de bis-imide(s) (a), de composé (b) et de composé organosilicique hydroxylé (c).

11°) Procédé selon l'une des revendications 2 à 10, caractérisé en ce que l'on utilise de 0,01 à 1 % et de préférence de 0,02 à 0,5 % en poids de composé imidazole par rapport à l'ensemble des réactifs (a) + (b) + éventuellement (c).

12°) Procédé selon l'une des revendications 2 à 11, caractérisé en ce que l'on utilise de 0 à 5 % et de préférence de 0,5 à 2 % en poids de N,N',N"-tris(hydroxyalkyl)hexahydrotriazine par rapport à l'ensemble de réactifs (a) + (b) + éventuellement (c).

13°) Procédé selon l'une des revendications 2 à 12, caractérisé en ce que l'on réalise un mélange des composés à groupements maléimides (a) et (b) et éventuellement du composé organosilicique hydroxylé (c), qui est fondu à une température allant de 50 °C à 300 °C, puis que l'on ajoute le composé imidazole (d), et le cas échéant la N,N',N"-tris-(hydroxylalkyl)hexahydrotriazine, éventuellement dissous dans un solvant, sous forte agitation, puis que l'on dégaze et que l'on coule rapidement la résine immédiatement après homogénéisation.

14°) Procédé de préparation sans faire appel à un composé organosilicique hydroxylé (c) selon l'une des revendications 2, 3, 5, 6, 8, 11 et 12, caractérisé en ce que l'on réalise d'abord un mélange intime du composé (b) et du composé imidazole (d), et le cas échéant de la N,N',N"-tris-(hydroxyalkyl)-hexahydrotriazine, éventuellement dissous dans un solvant, puis que l'on ajoute le composé (a) dans le mélange précité qui est chauffé à une température allant de 50 °C à 300 °C, puis que l'on dégaze et que l'on coule la résine immédiatement après homogénéisation.

15°) Procédé selon l'une des revendications 13 et 14, caractérisé en ce que le composé imidazole, et le cas échéant la N,N',N"-tris(hydroxyalkyl)hexahydrotriazine, sont dissous avant leur addition, dans du triallylisocyanurate, du phtalate de diallyle ou du benzoate d'allyle.